# EUROPÄISCHE PATENTSCHRIFT

(11) **EP 1 088 076 B1**
(45) Veröffentlichungstag und Bekanntmachung des Hinweises auf die Patenterteilung: **29.11.2006**
(21) Anmeldenummer: 00929370.5
(22) Anmeldetag: 17.04.2000
(51) Int. Cl.: C12N 15/36, C12N 1/06, C12N 1/19, C07K 14/02, C12R 1/78

(54) **VERFAHREN ZUM GEWINNEN VON REKOMBINANTEM HBsAg**
METHOD FOR OBTAINING RECOMBINANT HBsAg
PROCEDE DE PRODUCTION DE HbSAg RECOMBINE

(30) Priorität: 23.04.1999 DE 19918619
(43) Veröffentlichungstag der Anmeldung: 04.04.2001
(73) Patentinhaber: RHEIN BIOTECH GESELLSCHAFT FÜR NEUE BIOTECHNOLOGISCHE PROZESSE UND PRODUKTE MBH, 40595 Düsseldorf (DE)
(72) Erfinder: PIONTEK, Michael, D-45136 Essen (DE); WENIGER, Michael, D-47053 Duisburg (DE)
(74) Vertreter: Kahlhöfer, Hermann
(86) Internationale Anmeldenummer: PCT/EP2000/003476
(87) Internationale Veröffentlichungsnummer: WO 2000/065065

(56) Entgegenhaltungen:
- US-A- 3 983 008
- JANOWICZ Z A ET AL: "SIMULTANEOUS EXPRESSION OF THE S AND L SURFACE ANTIGENS OF HEPATITIS B AND FORMATION OF MIXED PARTICLES IN THE METHYLOTROPHIC YEAST HANSENULA-POLYMORPHA" YEAST, Bd. 7, Nr. 5, 1991, Seiten 431-444, XP000939178 ISSN: 0749-503X in der Anmeldung erwähnt
- DYR J E ET AL: "Separation used for purification of recombinant proteins" JOURNAL OF CHROMATOGRAPHY B: BIOMEDICAL SCIENCES & APPLICATIONS, Bd. 699, Nr. 1-2, 10. Oktober 1997 (1997-10-10), Seiten 383-401, XP004095005 ISSN: 0378-4347
- GELLISSEN G ET AL: "Application of yeasts in gene expression studies: a comparison of Saccharomyces cerevisiae, Hansenula polymorpha and Kluyveromyces lactis - a review" GENE: AN INTERNATIONAL JOURNAL ON GENES AND GENOMES, Bd. 190, Nr. 1, 29. April 1997 (1997-04-29), Seiten 87-97, XP004064388 ISSN: 0378-1119
- PITTROFF, M. ET AL.: "Mechanischer Aufschluss von Mikroorganismen im Verfahrensvergleich zwischen Nassvermahlung und Hochdruck-Homogenisation" CHEMIE. INGENIEUR. TECHNIK., Bd. 64, 1992, Seiten 950-953, XP002149036 ISSN: 0009-286X in der Anmeldung erwähnt
- BARATTI J ET AL: "PREPARATION AND PROPERTIES OF IMMOBILIZED METHANOL OXIDASE" BIOTECHNOLOGY AND BIOENGINEERING, Bd. 20, Nr. 3, 1978, Seiten 333-348, XP002149037 ISSN: 0006-3592

## Beschreibung

Die vorliegende Erfindung betrifft ein Verfahren zum Gewinnen von rekombinantem HBsAg aus HBsAg exprimierenden Hefezellen.

Die Zerstörung der Mikroorganismenzellwand - der Zellaufschluß - ist der erste Verfahrensschritt bei der Gewinnung intrazellulärer, biologisch aktiver Proteine. Im Produktionsmaßstab haben sich in den letzten Jahren hierfür besonders mechanische Aufschlußverfahren wie die Naßvermahlung in Rührwerkskugelmühlen bewährt.

Die schnellaufenden Rührwerkskugelmühlen geschlossener Bauart haben sich aufgrund der stetig gesteigerten Zerkleinerungs- und Dispergieranforderungen aus den konventionellen Kugelmühlen der Pigmentverarbeitung entwickelt. Sie bestehen aus einem um eine vertikale oder horizontale Achse rotierenden Hohlzylinder, der mit Glas- oder Zirkonium-Mischoxidkugeln bis zu einem Füllgrad von 80% befüllt ist. Dabei kann die Drehzahl zur Erhöhung der Prall-Druckzerkleinerung nicht beliebig erhöht werden da die Zentrifugalkräfte die Mahlwirkung aufheben. Des weiteren treten bei höheren Drehzahlen oder Füllgraden thermische Probleme auf, die das zu gewinnende Produkt schädigen können. Die kontinuierliche Mahlgut-Mahlkörper-Separierung erfolgt mit einer Siebpatrone, einem rotierenden Siebspalt oder mit einem im Lagergehäuse integrierten koaxialen Ringspalt. Die Kühlung des Mahlgutes erfolgt über einen Doppelmantel um den Mahlbehälter, teilweise kann die rotierende Achse ebenfalls gekühlt werden. Das Aufschlußprinzip der Naßvemahlung ist die Übertragung der Bewegungsenergie vom Rührwerk auf die Mahlkörper. Dies geschieht vorwiegend durch Adhäsionskräfte in Verbindung mit Verdrängungskräften, die auf die Anordnung und Ausführung der Rührelemente zurückzuführen sind. Hierdurch und durch Kohäsionskräfte wird der Mahlraum aktiviert. Durch die Beschleunigung der Mahlkörper in radialer Richtung kommt es zur Ausbildung einer Schichtenströmung. Die differentiellen Geschwindigkeitsprofile zwischen den Mahlkörperschichten bewirken dabei je nach Absolutgeschwindigkeit und Größe der bewegten Mahlkörper hohe Scherkräfte, die neben Kollisionsereignissen hauptverantwortlich für die Zerstörung der Mikroorganismenzellwand sind.

Zellwände lassen sich auch durch Hochdruckhomogenisatoren mechanisch aufbrechen Ein solches Gerät besteht im wesentlichen aus einer Hochdruck-Kolbenpumpe und einer Homogenisierungseinheit. Ein Homogenisierventil öffnet, wenn der eingestellte Druck erreicht wird, wobei dann die Zellsuspension mit sehr hoher Geschwindigkeit durch die Ventileinheit gedrückt wird. Die Zellsuspension erwärmt sich dabei um ca. 2,5°C pro 100 bar. Nach dem Verlassen der Aufschlußventileinheit wird die Zellsuspension über einen Wärmeaustauscher gekühlt. Anders als bei der Naßvermahlung tritt hier also eine kurzzeitige Erwärmung des Aufschlußgutes auf. Beim Passieren der Homogenisiereinheit wird die Zellsuspension sehr hohen Turbulenzen, Kavitation und Scherkräften ausgesetzt. Das Aufschlußprinzip eines Hochdruckhomogenisators ist der plötzliche Abbau der Energiedichte in der Zellsuspension in extrem kurzer Zeit, d.h. die hohe Druckdifferenz bzw. der schnelle Druckabfall kann als Hauptfaktor für den Aufschlußgrad angesehen werden.

Beide mechanische Aufschlußverfahren werden für verschiedene Mikroorganismen angewendet. Die Auswahl des jeweiligen Verfahrens hängt von der Art des Mikroorganismus, insbesondere von seiner Struktur ab. So werden beispielsweise in Pittroff, M. et al., DECHEMA-Biotechnol. Conf.; (1990), 4, Pt.B, 1055-60 beide Zelltrümmerungsverfahren für verschiedene Mikroorganismen, wie *Saccharomyces cerevisiae, Micrococcus luteus* und *Escherichia coli,* getestet, wobei geschlußfolgert werden konnte, daß morphologische Unterschiede der Mikroorganismen die Trümmerleistung der beiden Verfahren beeinflussen.

So verweisen Pittroff, M. et al., DECHEMA-Biotechnol. Conf.; (1992), 5, Pt.B, 687-91 und Pittroff, M. et al., Chem. lng. Tech.; (1992), 64, 10, 950-53 darauf, daß für bestimmte Mikroorganismen, wie stabförmige Bakterien, bevorzugt das Hochdruckaufschlußverfahren durchgeführt wird. Dagegen wird für Kokken, die eine runde Struktur aufweisen, vorzugsweise das Rührwerkskugelmühlen-Verfahren eingesetzt. Bei einem Aufschluß von Hefezellen der Spezies *Saccharomyces cerevisiae* wird für beide Verfahren der gleiche Desintegrationsgrad beobachtet.

Weiterhin beschreiben Luther, H. et al., Acta - Biotechnol.; (1992), 12, 4, 281-91 einen weiteren Vergleich der Rührwerkskugelmühlen- und der Hochdruckhomogenisatoren zur Reinigung von Protein aus *Saccharomyces cerevisiae* oder *Escherichia coli.* Es wird bestätigt, daß sowohl Hefe- als auch Bakterienzellen mit Rührwerkskugelmühlen und mit Hochdruckhomogenisatoren aufgeschlossen werden können. Als ein Nachteil der Hochdruckhomogenisatoren wird herausgestellt, daß schleimbildende Mikroorganismen oder mechanische Kontaminationen sehr leicht zu Okklusionen führen. In bezug auf Hefezellen wird festgestellt, daß der Hochdruckhomogenisator weniger Zellen zerstört. Obwohl dies nicht ausdrücklich erwähnt worden ist, muß daher von einer geringeren Ausbeute mittels Hochdruckhomogenisator ausgegangen werden.

In Schütte, H., Biol. Recombinant-Microorg. Anim. Cells: (1991) Oholo 34 Meet., 293-305 wird ebenfalls bekräftigt, daß Hochdruckhomogenisatoren einen wirksamen Zellaufschluß bei Hefe- und Bakterienzellen bewirken, jedoch für Mycel-bildende Organismen nicht geeignet sind.

In Baldwin, C., Biotechnol. Tech.; (1990), 96, 4, 329-34 wurde der Aufschluß von *Saccharomyces cerevisiae* mit einem Hochdruckhomogenisator untersucht. Dabei wurde herausgefunden, daß das Aufbrechen der Zellen mittels Hochdruckhomogenisator im allgemeinen nur zu niedrigen Zertrümmerungsausbeuten führt (40% in 5 Passagen). Ein völliger Aufschluß der Hefezellen im Hochdruckhomogenisator konnte nur beobachtet werden, wenn vorher eine Enzymbehandlung mit Zymolase aus *Oerskovia xanthineolytica* durchgeführt worden war.

Aus den umfangreichen Untersuchungen, die bisher durchgeführt wurden, ist weiterhin ersichtlich, daß die Art des zu reinigenden Proteins von einer entscheidenden Bedeutung für die Auswahl des jeweiligen Aufschlußverfahrens ist. Insbesondere ist dabei der Zellaufschluß von Hepatitis B Oberflächen-Antigen (HBsAg) exprimierenden Zellen von Interesse.

In Choo, K.B. et al., Biochem. Biophys. Res. Commun.; (1985), 131, 1, 160-66 wird der Einsatz von Rührwerkskugelmühlen für den Aufschluß von *Saccharomyces cerevisiae-*Zellen zur Gewinnung von HBsAg untersucht. Es konnte jedoch nur eine geringe Menge an HBsAg in partikulärer Form erhalten werden.

Fenton, D.M. et al., Abstr. Ann. Med. Am. Soc. Microbiol.; (1984), 84 Meet. 193 beschreiben die Freisetzung von rekombinantem HBsAg aus *Saccharomyces cerevisiae* mittels Zellaufschluß in einem Hochdruckhomogenisator, in dieser Literaturstelle wird darauf hingewiesen, daß die Solubilisierung von HBsAg in aktiver, d.h. antigener Form im großen Maßstab problematisch ist. Ein ausreichender Zellaufschluß und zufriedenstellende Proteinfreisetzung wurden in einem Hochdruckhomogenisator erst nach 10 Passagen beobachtet, und eine maximale HBsAg-Freisetzung konnte sogar erst nach 15 Passagen erhalten werden. Die Autoren schlossen daraus, daß die Freisetzung von antigenem HBsAg die Zertrümmerung subzellulärer Strukturen erfordert.

Demzufolge konnten bisher mit den beiden Aufschlußverfahren noch keine geeigneten Systeme zur optimalen Herstellung von HBsAg durch Zellaufschluß zur Verfügung gestellt werden.

Es ist deshalb die Aufgabe der vorliegenden Erfindung, ein Verfahren zu entwickeln, mit dem rekombinantes HBsAg in hoher Ausbeute aus rekombinanten Mikroorganismen gewonnen werden kann.

Diese Aufgabe wird erfindungsgemäß gelöst durch ein Verfahren zum Gewinnen von rekombinantem HBsAg, bei dem zur Expression von HBsAg fähige rekombinante methylotrophe Hefezellen unter Verwendung eines Hochdruckhomogenisators aufgeschlossen werden, und das HBsAg aus den erhaltenen Zelltrümmern gewonnen wird.

Es wurde überraschend gefunden, daß beim Zellaufschluß von HBsAg exprimierenden *Hansenula polymorpha*-Zellen in einem Hochdruckhomogenisator eine bedeutend höhere Produktausbeute pro g Zelltrockengewicht erreicht werden konnte als mit den herkömmlichen Verfahren, insbesondere dem Zellaufschluß aus *Saccharomyces cerevisiae* mittels Hochdruckhomogenisator oder Glaskugelmühlen. Das erfindungsgemäße Verfahren stellt daher eine erhebliche Verbesserung gegenüber den bisherig bekannten Verfahren zur Gewinnung von HBsAg aus Mikroorganismen dar.

In dem erfindungsgemäßen Verfahren wird rekombinantes HBsAg aus rekombinanten methylotrophen Hefezellen gewonnen, die zur Expression von HBsAg in der Lage sind. Vorzugsweise wird ein HBsAg exprimierender Stamm der Gattung *Hansenula* verwendet, besonders bevorzugt *Hansenula polymorpha.* Methylotrophe Hefen der Gattungen *Pichia, Candida und Torulopis* können ebenfalls verwendet werden. Während der Fermentation werden die üblichen Parameter, wie pH, Belüftung und Temperatur kontrolliert. Als Substrat für die Hefezellen sind Glycerin, Methanol und Glucose, vorzugsweise Glycerin, geeignet. Das Substrat wird der Fermenterkultur zugeführt, bis die Hefezellen eine gewünschte Zelldichte von mindestens 80, vorzugsweise mindestens 90 g·l⁻¹ Zelltrockengewicht erreicht haben. Wenn diese Zelldichte erreicht wird, wird die Expression des HBsAg in der Hefekultur induziert.

In bevorzugten Ausführungsformen wird die Expression des HBsAg in der methylotrophen Hefe durch einen Promotor kontrolliert, der einem in den Methanolmetabolismus involviertem Gen entstammt. Gut beschriebene Promotoren sind der MOX-Promotor, der DAS- und der FMD-Promotor (Ledeboer, A.M. et al., Nucl. Acids Res. (1985), 13, 9, 3063-3082; Janowicz, Z.A. et al., Nucl. Acids Res. (1995), 13, 9, 2043-3062, EP 299 108). Um eine optimale Expression unter der Kontrolle dieser Promotoren zu erzielen, werden die Hefezellen zunächst in vollsynthetischem Nährmedium kultiviert. Zur Induktion wird Methanol zugefügt, so daß die Zellsuspension ca. 1 %-ig an Methanol ist. Genaue Angaben für die Kultivierung von methylotrophen Hefen und die Induktionsbedingungen für die drei genannten Promotoren können z.B. in Gelissen, G. in Murooka/lmanaka (eds.) Recombinant microbes for industrial and agricultural applications, Marcel Dekker, NY 1993, 787-796 und Weydemann, U. et al., Appl. Microbiol. Biotechnol. (1995), 44, 377-385 gefunden werden.

Nach Beendigung der Fermentation werden die Zellen von den Medienbestandteilen über Tangentialfluß-Filtration separiert. Durch geeignete Verdünnung mit einem Aufschlußpuffer wird eine gewünschte Zelldichte eingestellt.

Zum Aufschluß werden die Hefezellen vorzugsweise bei einer Zelldichte von 50 bis 150 g·l⁻¹ Zelltrockengewicht verwendet. Die genaue Zelldichte ist abhängig von der jeweiligen rekombinanten methylotrophen Hefezellengattung, insbesondere ist eine Zelldichte von 70 bis 120 g·l⁻¹ Zelltrockengewicht bevorzugt. Wird die Zelldichte der erfindungsgemäß einzusetzenden rekombinanten methylotrophen Hefezellen zu niedrig gewählt, wird das Verfahren zu zeitaufwendung und unökonomisch. Bei einer zu hohen Zelldichte der Hefezellen wird der Aufschluß zunehmend ineffizienter, und es steigen die Anforderungen an die Kühlleistung.

Bei dem erfindungsgemäß verwendeten Hochdruckhomogenisator handelt es sich um einen, wie in der Einleitung beschriebenen, herkömmlichen Hochdruckhomogenisator, der zum Zellaufschluß verwendet werden kann. Es ist besonders bevorzugt, daß ein Hochdruckhomogenisator vom Typ Nanojet LAB30 PL oder ein vergleichbares Modell verwendet wird. Während des Zellaufschlusses beträgt der Druck in der Homogenisiereinheit 1000 bis 2000 bar. Ein Druck von 1200-1600 bar ist bevorzugt. Besonders bevorzugt ist ein Druck von 1500-1600 bar.

Die Ausgangstemperatur der Hefezellen beträgt vorzugsweise 2 bis 15°C. insbesondere bevorzugt 4 bis 8°C. Die Hefezellen, die üblicherweise in Form einer Zellsuspension vorliegen, können unter Rühren auf die gewünschte Temperatur gekühlt werden. Der Hochdruckhomogenisator wird vorzugsweise gekühlt, so daß sich am Produktauslaß eine niedrige Auslaßtemperatur von beispielsweise 2 bis 15°C, vorzugsweise 3 bis 13°C. besonders bevorzugt 5 bis 10°C, eingestellt.

Im Hochdruckhomogenisator werden die Zellen üblicherweise in mehreren Zyklen (Passagen) aufgeschlossen. Es hat sich gezeigt, daß insgesamt 3 bis 8 Zyklen (Passagen) für eine optimalen Produktausbeute ausreichend sind. Bevorzugt werden 3 bis 6, besonders bevorzugt 4 Zyklen (Passagen) für den Zellaufschluß zum Gewinnen von rekombinantem HBsAg verwendet.

Nach Beendigung des Zellaufschlusses wird die so erzeugte Präparation vorzugsweise weiteren Separations- und Reinigungsschritten unterzogen. Diese zusätzlichen Prozeßschritte sind herkömmliche Verfahren, die eine weitere Reinigung und Anreicherung von rekombinantem HBsAg im Extrakt bewirken. So können einer oder mehrere der folgenden Separations- und Aufreinigungsschritte, beispielsweise in der angegebenen Reihenfolge, durchgeführt werden:

Fällung der Zelltrümmer mit Polyethylenglycol, Separation des PEG-Überstandes, Adsorption an eine Silika-Matrix, Separation der Silika-Matrix, Desorption des Produkts von der Silika-Matrix , Separation des Überstandes der Silika-Matrix, lonenaustauschchromatographie, Konzentrierung des lonentauscher-Pools mit Hilfe einer Ultrafiltration, Dichtegradiententrennung in Cäsiumchlorid, Größenausschlußchromatographie und Sterilfiltration (Final Aqueous Bulk).

Das nachstehende Beispiel erläutert die Erfindung.

### Beispiel 1

### Zellaufschluß von Hansenula polymorpha zum Gewinnen von HBsAg durch Hochdruckaufschluß

### 1. Hochdruckhomogenisator

Für das Hochdruckaufschlußverfahren wurde ein Gerät folgender Spezifikationen verwendet:

**Nanojet Lab30 PL:**

| | |
|---|---|
| Aufschlußprinzip: | Hochdruck |
| Volumenstrom: | (100 ± 20) ml·min.⁻¹, nicht regulierbar |
| Eingangsdruck Kolbenpumpe: | 4 - 7 bar |
| Druck Homogenisiereinheit: | 1200 -1600 bar |
| Homogenisierventil: | Wolfram-Carbid |
| Kühlung: | Rohrbündel-Wärmetauscher |
| Zelltrockengewicht: | 70 g-l⁻¹ |
| Dichtungen: | EPDM |
| Hochdruckdichtung: | Buna N |
| Material: | 1-45-71 Stahl |

### 2. Mikroorganismus und Kultur desselben

Ein HBsAg exprimierender *Hansenula polymorpha* Stamm, z.B. der in Janowicz et al., Yeast, 7:431-443, 1991 beschriebene Stamm wird in einem vollsynthetischen Nährmedium kultiviert. Während der Fermentation werden pH, Belüftung und Temperatur kontrolliert. Der Fermenterkultur wird während der Fermentation in Abhängigkeit von der Konzentration an gelöstem Sauerstoff absatzweise Glycerin als Substrat bis zu einer Zelldichte von ca. 90 g·l⁻¹ Zelltrockengewicht zugeführt. Ab einer Zelldichte von ca. 90 g·l⁻¹ Zelltrockengewicht und einer Fermentationszeit von ca. 46 Stunden wird die Expression des HBsAg in der *Hansenula polymorpha* Kultur durch die einmalige Zugabe von Methanol innerhalb von 18 bis 26 Stunden induziert. Das Zelltrockengewicht wird aus einem Kulturaliquot bestimmt, indem auf einer Trockengewichtswaage bis zur Gewichtskonstanz eingedampft wird. Nach der letzten Zugabe wird die Kultur für weitere 2 bis 9 Stunden kultiviert. Anschließend werden die Zellen durch Cross-Flow-Filtration weitgehend von Fermentationsmedienbestandteilen befreit. Vor Aufschluß werden die Zellen umgepuffert. Die Umpufferung findet dabei mit 20 mM Phosphatpuffer sowie 2 mM Na₂-Ethyleniamintetraessigsäure·2H₂O (Titriplex III) statt. Der finalen Zellsuspension werden 75 mg Detergens *Tween* _{*2*}*0* pro g Zelltrockengewicht zugeführt. Zum Aufschluß wurden *Hansenula polymorpha* Fermenterkulturbrühen mit einer Zelldichte von ca. 70 g·l⁻¹ Zelltrockengewicht verwendet.

### 3. Zellaufschluß

Die umgepufferten Zellen werden unter Rühren auf 4 - 8°C gekühlt. Die Ausgangstemperatur am Aufschlußgerät wird auf 8 ± 5°C eingestellt. Der Zellextrakt wird in einem separaten gekühlten Rührbehälter aufgefangen.

Der Volumenstrom beim Hochdruckhomogenisator wird durch das Kolbenhubvolumen sowie der Anzahl der Hübe pro Minute bestimmt und kann nicht individuell eingestellt werden. In der Regel wird bei bei einer Flußrate von 100 ml Zellsuspension pro Minute aufgeschlossen. Die Zellen werden in insgesamt vier Zyklen aufgeschlossen. Nach beendetem Zyklus 4 wird mit 80 mM PMSF-Stammlösung auf 2 mM Endkonzentration eingestellt.

Nach dem Zellaufschluß kann das Produkt durch folgende Separations- und Aufreinigungsschritte in der genannten oder einer anderen Reihenfolge gereinigt werden:
- Fällung der Zelltrümmer mit Polyethylenglycol
- Separation des PEG-Überstandes
- Adsorption an eine Silika-Matrix
- Separation der Silika-Matrix
- Desorption des Produkts von der Silika-Matrix
- Separation des Überstandes der Silika-Matrix
- lonenaustauschchromatographie
- Konzentrierung des lonentauscher-Pools mit Hilfe einer Ultrafiltration
- Dichtegradiententrennung in Cäsiumchlorid
- Größenausschlußchromatographie
- Sterilfiltration (Final Aqueous Bulk)

Um die Effizienz des Hochdruckaufschlusses zu untersuchen, wird der Gesamtproteingehalt mit Hilfe der Lowry-Methode, sowie die Konzentration an HBsAg mit einem immunologischen Verfahren ("ELISA") in den Zellextrakten ermittelt.

Im Nanojet Hochdruckaufschluß wurde im 101-Fermentationsmaßstab gearbeitet. Um Vergleichbarkeit zu gewährleisten, wird die Ausbeute an HBsAg auf die jeweilige Zellmasse vor dem Zellaufschluß bezogen.

### 4. Ergebnisse

### 4. 1 Austestung des Nanojet Hochdruckaufschlusses

- Stamm:: *Hansenula polymorpha* K3/8-1
- Ausgangsmaterial:: 10 Liter Fermenterkultur RL-98/17
- Ziel:: Festlegung der notwendigen Anzahl der Aufschlußzyklen

| **Passage** | **Gesamtprotein [mg]** | **Gesamt-HBsAg (Produkt) [mg]** |
|---|---|---|
| NJ-2-**1** | 2208 | 118 |
| NJ-2-**2** | 3898 | 278 |
| NJ-2-**3** | 4968 | 340 |
| NJ-2-**4** | 4884 | 440 |
| NJ-2-**5** | 4795 | 430 |
| NJ-2-**6** | 5088 | 434 |

Ab der vierten Passage konnte kein signifikanter Zuwachs an Protein und Produkt (HBsAg) mehr festgestellt werden. Für die weiteren Versuche wurde daher eine Passagenzahl von vier festgelegt.

### 4.2 Aufschlußeffizienz von Nanojet Hochdruckaufschluß für Rohextrakt und "Final Aqueous Bulk" (Endprodukt)

Der "Final Aqueous Bulk" ist das Präparat, das nach der Durchführung der vorstehend genannten Separations- und Aufbereitungsschritte erhalten wird.
- Stamm:: *Hansenula polymorpha* K3/8-1
- Ausgangsmaterial:: 10 Liter Fermenterkultur NJ-4 () RL-98/19), NJ-6, -7

| | **mg Protein/g Zelltrokkengewicht** | | **mg HBsAg/g Zelltrockengewicht** | |
|---|---|---|---|---|
| Nanojet LAB30PL | Rohextrakt | Final Aqueos Bulk | Rohextrakt | Final Aqueous Bulk |
| Fermentation NJ-4 | 128,2 | 1,98 | 10,8 | 1,77 |
| Fermentation NJ-6 | 100,4 | 1.29 | 7,1 | 1,41 |
| Fermentation NJ-7 | 92,6 | 0,99 | 6,9 | 1,10 |
| **Durchschnitt** | **107,1** | **1,42** | **8,3** | **1,43** |

### Vergleichsbeispiel 1

### Zellaufschluß von Hansenula polymorpha zum Gewinnen von HBsAq durch Naßvermahlung

### 1. Glaskugelhomogenisator

Für das Naßvermahlungsverfahren wurden Geräte folgender Spezifikationen verwendet.

**Fryma Coball Mill MS18:**

| | |
|---|---|
| Aufschlußprinzip: | Naßvermahlung |
| Rotordurchmesser: | 192 mm |
| effektives Mahlvolumen: | 1200 ml |
| Befüllung mit Mahlkörpern: | 70 - 80% des effektiven Mahlvolumens |
| Mahlspalt: | 6,50 mm |
| Trennspalt: | 0,05 mm |
| Siebspalt: | 0,20 mm |
| Differentialspalt: | 0,18 mm |
| Rotorumfangsgeschwindigkeit: | 14 m·s⁻¹ |
| Kühlung: | Doppelmantel und Rotorkühlung |
| Zelltrockengewicht: | 100 - 120 g·l⁻¹ |
| Volumenstrom: | 200 - 300 ml·min⁻¹ |
| Dichtungsmaterial: | Ethylen-Propylen |

**Dyno Mill KDL Spezial:**

| | |
|---|---|
| Aufschlußprinzip: | Naßvermahlung |
| effektives Mahlraumvolumen: | 600 ml |
| Rührscheiben: | 4, Polyurethan |
| Befüllung mit Mahlkörpern: | 80 - 85% des effektiven Mahlvolumens |
| Umdrehungsgeschwindigkeit: | 6,8 m·s⁻¹ |
| Koaxialer Ringspalt: | 0,1 mm |
| Kühlung: | Doppelmantel |
| Zelltrockengewicht: | 70 g·l⁻¹ |
| Volumenstrom: | 100 ml·min⁻¹ |
| Dichtungsmaterial: | Viton |

Zur Naßvermahlung wurden Glaskugeln mit 0,45 - 0,55 mm Durchmesser verwendet.

### 2. Mikroorganismus und Kultur desselben

Es werden dieselben Materialien wie in Beispiel 1 werden verwendet, jedoch besitzen die Fermenterkulturbrühen eine Zelldichte von 70 bis 120 g·l⁻¹ Zelltrockengewicht.

### 3. Zellaufschluß

Die Vor- und Nachbehandlung erfolgen wie in Beispiel 1 beschrieben.

### Naßvermahlung in der Dyno Mill KDL Spezial

Die horizontal ausgerichtete Aufschlußkammer wird über eine Peristaltikpumpe mit einem Volumenstrom von 100 ml·min.⁻¹ Zellsuspension, die 70 g·l⁻¹ Zellen enthält, kontinuierlich beschickt. Zeitgleich wird eine 80 mM PMSF-Lösung zur Proteaseinhibierung mit 1/40 des Zellsuspensionstroms an einem separaten Eingangsport der Aufschlußkammer zugegeben, wobei eine sofortige Vermischung erfolgt. Die Zellsuspension wird in einem Zyklus aufgeschlossen.

### Naßvermahlung in der Fryma Coball Mill MS 18

Hier wird die Zellsuspension der Aufschlußkammer, die vertikal ausgerichtet ist, ebenfalls mit einer Peristaltikpumpe kontinuierlich zugeführt. Jedoch beträgt die Zelldichte in diesem Fall 100 bis 120 g·l⁻¹, der Volumenstrom wird auf 200 bis 300 ml·min.⁻¹ eingestellt. Die Zugabe der Proteaseinhibitor-Stammlösung (80 mM PMSF) erfolgt im Gegensatz zur Dyno Mill KDL Spezial erst am Kugelmühlenauslaß. Die Zellsuspension wird in einem Zyklus aufgeschlossen. Die Endkonzentration an PMSF im finalen Zellextrakt beträgt 2 mM.

Die Aufschlußversuche mit der Fryma Coball Mill MS18 wurden im 50 Liter Maßstab durchgeführt. Bei der Naßvermahlung mit der Dyno Mill KDL Spezial wurde im 101-Fermentationsmaßstab gearbeitet. Um Vergleichbarkeit zu gewährleisten, wird die Ausbeute an HBsAg auf die jeweilige Zellmasse vor dem Zellaufschluß bezogen.

### 4. Ergebnisse

### 4.1 Aufschlußeffizienz im Dyno Mill Glaskugelaufschluß

- Stamm:: *Hansenula polymorpha* K3/8-1
- Ausgangsmaterial:: 10 Liter Fermenterkultur RL-98/19

| **Aufschlußgerät** | **Prozeßschritt** | **mg** _{Protein} **/g** _{Zelltrockengewicht} **(Lowry)** | **mg** _{**HBsAg**}**/g** _{Zelltrockengewicht} **(ELISA)** |
|---|---|---|---|
| Dyno Mill KDL | Rohextrakt | 69,4 | 2,35 |

### 4.2 Aufschlußeffizienz im Fryma-Glaskugelaufschluß bis zum Endprodukt "Final Aqueous Bulk"

- Stamm:: *Hansenula polymorpha* K3/8-1
- Ausgangsmaterial:: 50 Liter Fermenterkultur RL-98/26, /29,/35

| | **mg Protein/g Zelltrockengewicht** | | **mg HBsAg/g Zelltrockengewicht** | |
|---|---|---|---|---|
| Fryma Coball Mill MS18 | Rohextrakt | Final Aqueous Bulk | Rohextrakt | Final Aqueous Bulk |
| Fermentation RL-98/26 | 75,7 | 0,56 | 2,85 | 0,53 |
| Fermentation RL-08/29 | 78,5 | 0,53 | 3,00 | n.d. |
| Fermentation RL-98/35 | 92,7 | 0,55 | 2,86 | 0,55 |
| **Durchschnitt** | **82,3** | **0,55** | **2,90** | **0,54** |

### Beispiel 2

### Vergleich zwischen Naßvermahlung und Hochdruckaufschluß

### 1. Aufschlußeffizienz von Nanojet Hochdruckaufschluß und Dyno Mill Glaskugelaufschluß

Verglichen mit dem Zellaufschluß in der DynoMill Glaskugelmühle wird aus identischem Ausgangsmaterial eine vierfach höhere Produktmenge pro Gramm Trockensubstanz freigesetzt. Die Gesamtproteinmenge steigt gleichzeitig nur um den Faktor zwei an. Es wird folglich mehr Produkt (HBsAg) aus dem gleichen Ausgangsmaterial freigesetzt. Die anfängliche Produktausbeutesteigerung im Hochdruckaufschlußverfahren bleibt auch nach allen folgenden Prozeßschritten bis hin zum Final Aqueous Bulk erhalten und die Produktqualität entspricht der des Standardverfahrens (Naßvermahlung). Dies konnte mittels der beschriebenen Kombination aus Lowry und ELISA gezeigt werden.

### 2. Aufschlußeffizienz von Nanojet Hochdruckaufschluß und Fryma Coball Mill Glaskugelaufschluß

Über den Durchschnitt von jeweils drei Läufen betrachtet wird mit dem Nanojet Hochdruck-Aufschluß eine 2,9-fach höhere Produktmenge pro Gramm Zelltrockengewicht freigesetzt als beim Glaskugelaufschluß. Die höhere Produktmenge aus dem Zellaufschluß führt zu einer 2,6-fach höheren Menge an aufgereinigtem Produkt im Final Aqueous Bulk. Die Qualität der Produkte liegt für beide Aufschlußverfahren innerhalb derselben Spezifikationen, wie auch die folgenden Analysenzertifikate bestätigen.

### 3. Analysenergebnisse

### 3.1 Hochdruckaufschluß

Probe: rHBsAg
Chargen-Nr. **NJ6** GFC

| Test | Ergebnisse | RB Spezifikation¹ | Bemerkungen |
|---|---|---|---|
| Proteingehalt Lowry | 3,96 mg/ml | | |
| Proteinreinheit SDS-PAGE/ kolloidale Comassie-Färbung | >95% rein | ≥095% | positiv |
| Identität Western blot | reagiert | reagiert mit Anti-HBsAg Antikörpern ! | positiv |
| Reaktivität | 6,61 mg/ml | | positiv |
| AUSZYME | 1,67 mg/mg | >1 mg/mg Protein | |
| | Protein | | |
| Lipidgehalt | 2,43 mg/ml | | positiv |
| Merckotest | 0,61 mg/mg | 0,5-1,8 mg Lipid/ | |
| | Protein | 1.0 mg Protein | |
| DNA-Gehalt | 1221 pg DNA/ml | | positiv |
| Schwellenwert | 6,1 pg/20 µg | <100 pg/20 µg Protein | |
| | Protein | | |
| Cäsiumgehalt | 8,7 ng/ml | | positiv |
| HR-ICP-MS² | 43,5 pg/20 µg | <10 µg/20 µg Protein | |
| | Protein | | |
| Kohlenhydratgehalt | 101 µg/ml | | positiv |
| Orcinol | 25,5 µg/mg Protein | <50 µg/mg Protein | |
| Endotoxin-Gehalt | 0,16 EU⁴/ml | | positiv |
| LAL³ | 0,04 EU/mg | <100 EU/mg Protein | |
| | Protein | | |

| | | | |
|---|---|---|---|
| ¹ festgelegte Grenzwerte unter Bezug auf Angaben der WHO (World Health Organisation) und der Europäischen Pharmacopeia ² High resolution-inductive coupled plasma - mass spectrometry ³ Limulus Amoebozyten Lysat (Testmethode zum Nachweis von Endotoxin) ⁴ Endotoxin Units | | | |

### 3.2 Glaskugelaufschluß

Probe: rHBsAg
Chargen-Nr. RL98/35 Final Bulk

| Test | Ergebnisse | RB Spezifikation¹ | Bemerkungen |
|---|---|---|---|
| | | | |
| Proteingehalt Lowry | 1,39 mg/ml | 1.0 - 2,0 mg/ml | positiv |
| Proteinreinheit SDS-PAGE/ kolloidale Comassie-Färbung | >95% rein | ≥95% | positiv |
| Identität Western blot | reagiert | reagiert mit Anti-HBsAg Antikörpern | positiv |
| Reaktivität | 1,92 mg/ml | | positiv |
| AUSZYME | 1,38 mg/mg | >1 mg/mg Protein | |
| | Protein | | |
| Lipidgehalt | 1,13 mg/ml | | positiv |
| Merckotest | 0,81 mg/mg | 0,5-1,8 mg Lipid/ | |
| | Protein | 1.0 mg Protein | |
| DNA-Gehalt | 56,4 pg DNA/ml | | positiv |
| Schwellenwert | 0,8 pg/20 µg | <100 pg/20 µg Protein | |
| | Protein | | |
| Cäsiumgehalt | 8,2 ng/ml | | positiv |
| HR-ICP-MS² | 118 pg/20 µg | <10 µg/20 µg Protein | |
| | Protein | | |
| Kohlenhydratgehalt | 35,8 µg/ml | | positiv |
| Orcinol | 26 µg/mg Protein | <50 µg/mg Protein | |
| Endotoxin-Gehalt | 0,75 EU/ml | | positiv |
| LAL³ | 0,54 EU/mg | <100 EU/mg Protein | |
| | Protein | | |

| | | | |
|---|---|---|---|
| ¹ festgelegte Grenzwerte unter Bezug auf Angaben der WHO (World Health Organisation) und der Europäischen Pharmacopeia ² High resolution-inductive coupled plasma - mass spectrometry ³ Limulus Amoebozyten Lysat (Testmethode zum Nachweis von Endotoxin) ⁴ Endotoxin Units | | | |

### 4. Schlußfolgerung

Die gezeigten Untersuchungen belegen, daß entgegen der im Stand der Technik wiedergegebenen Annahme der Hochdruckaufschluß für Hefezellen, jedenfalls für Hefezellen methylotropher Gattungen, von erheblichem Vorteil ist. Mittels Hochdruckaufschluß kann eine Steigerung der Ausbeute um das 2,5- bis 4-fache erreicht werden.

## Patentansprüche

1. Verfahren zum Gewinnen von rekombinantem Hepatitis B Oberflächen-Antigen, bei dem zur Expression von Hepatitis B Oberflächen-Antigen fähige rekombinante methylotrophe Hefezellen unter Verwendung eines Hochdruckhomogenisators aufgeschlossen werden und das Hepatitis B Oberflächen-Antigen aus den erhaltenen Zelltrümmern gewonnen wird.

2. Verfahren nach Anspruch 1, **dadurch gekennzeichnet, daß** die rekombinante methylotrophe Hefezelle eine Hefezelle der Gattung *Hansenula* ist.

3. Verfahren nach Anspruch 1 oder 2, **dadurch gekennzeichnet, daß** die Hefezellen bei einer Zelldichte von 50 -150 g • l⁻¹ Zelltrockengewicht für den Aufschluß verwendet werden.

4. Verfahren nach einem der Ansprüche 1 bis 3, **dadurch gekennzeichnet, daß** die Hefezellen bei einem Druck von 1200 bis 1600 bar in der Homogenisiereinheit des Hochdruckhomogenisators aufgeschlossen werden.

5. Verfahren nach einem der Ansprüche 1 bis 3, **dadurch gekennzeichnet, daß** die Temperatur am Produktauslaß 2 bis 15°C beträgt.

6. Verfahren nach einem der Ansprüche 1 bis 5, **dadurch gekennzeichnet, daß** die Zellen in 3 bis 6 Zyklen (Passagen) aufgeschlossen werden.

7. Verfahren nach einem der Ansprüche 1 bis 6, **dadurch gekennzeichnet, daß** das rekombinante Hepatitis B Oberflächen-Antigen nach dem Zellaufschluß weiteren Separations- und Reinigungsschritten unterzogen wird.

## Claims

1. Process for obtaining recombinant hepatitis B surface antigen, in which recombinant methylotrophic yeast cells capable of expression of hepatitis B surface antigen are disrupted by using a high pressure homogeniser and the hepatitis B surface antigen is obtained from the obtained cell debris.

2. Process according to claim 1, **characterised in that** the recombinant methylotrophic yeast cell is a yeast cell of the species *Hansenula.*

3. Process according to claim 1 or claim 2, **characterised in that** the yeast cells are used for the disruption with a cell density of 50 - 150 g · 1⁻¹ cell dry weight.

4. Process according to any one of claims 1 to 3, **characterised in that** the yeast cells are disrupted at a pressure of 1200 to 1600 bar in the homogenisation unit of the high pressure homogeniser.

5. Process according to any one of claims 1 to 3, **characterised in that** the temperature at the product outlet is 2 to 15 °C.

6. Process according to any one of claims 1 to 5, **characterised in that** the cells are disrupted in 3 to 6 cycles (passages).

7. Process according to any one of claims 1 to 6, **characterised in that** the recombinant hepatitis B surface antigen after the cell disruption is subjected to further separation and purification steps.

## Revendications

1. Procédé pour obtenir des antigènes de surface recombinants d'hépatite B, dans lequel des cellules de levure méthylotrophes recombinantes, capables d'expression de l'antigène de surface d'hépatite B, sont décomposées en utilisant un homogénéisateur à haute pression et l'antigène de surface d'hépatite B est obtenu à partir des débris de cellules obtenus.

2. Procédé selon la revendication 1, **caractérisé en ce que** la cellule de levure méthylotrophe recombinante est une cellule de l'espèce *Hansenula.*

3. Procédé selon la revendication 1 ou 2, **caractérisé en ce que** pour la décomposition les cellules de levure sont utilisées à une densité cellulaire de 50-150 g · 1⁻¹ poids sec cellulaire.

4. Procédé selon l'une des revendications 1 à 3, **caractérisé en ce que** les cellules de levure sont décomposées à une pression de 1200 à 1600 bar dans l'unité d'homogénéisation de l'homogénéisateur.

5. Procédé selon l'une des revendications 1 à 3, **caractérisé en ce que** la température à la sortie du produit comporte 2 à 15 °C.

6. Procédé selon l'une des revendications 1 à 5, **caractérisé en ce que** les cellules sont décomposées en 3 à 6 cycles (passages).

7. Procédé selon l'une des revendications 1 à 6, **caractérisé en ce qu'**après la décomposition cellulaire l'antigène de surface recombinant d'hépatite B est soumis à d'autres étapes de séparation et de purification.
